# EUROPEAN PATENT APPLICATION

(11) **EP 1 790 351 A1**
(43) Date of publication of application: **30.05.2007**
(21) Application number: 05782391.6
(22) Date of filing: 12.09.2005
(51) Int. Cl.: A61K 38/00, A61K 38/21, A61K 47/36

(54) **PERMUCOSAL COMPOSITION AND METHOD OF IMPROVING PERMUCOSAL ABSORPTION**

(30) Priority: 15.09.2004 JP 2004268891
(71) Applicant: Otsuka Pharmaceutical Company, Limited, Tokyo 101-8535 (JP)
(72) Inventor: YAMAMOTO, Akira Pakuhausu Kyoto Okazaki, Kyoto-shi, Kyoto 6068344 (JP); YAMADA, Keigo OTSUKA PHARMACEUTICAL CO., LTD., Tokushima-shi (JP)
(74) Representative: HOFFMANN EITLE
(86) International application number: PCT/JP2005/016739
(87) International publication number: WO 2006/030730

(57) **Abstract**

An object of the invention is to provide a composition for transmucosal administration that enables pharmacologically active peptides and proteins to be efficiently absorbed through the mucosa such as the pulmonary mucosa, nasal mucosa, oral mucosa, vaginal mucosa, gastric mucosa, gastrointestinal mucosa or the like.

A composition for transmucosal administration are prepared by adding (i) at least one member selected from the group consisting of peptides and proteins having pharmacological activity; and (ii) at least one member selected from the group consisting of chitosan oligosaccharides having a polymerization degree of from 2 to 20, derivatives thereof, glucosamine, and salts thereof.

## Description

### TECHNICAL FIELD

The present invention relates to a composition for transmucosal administration, and more specifically a composition for transmucosal administration enabling improved transmucosal absorption of pharmacologically active peptides and proteins. Also, the present invention relates to a method of enhancing the transmucosal absorption of pharmacologically active peptides and proteins.

### BACKGROUND ART

Conventionally, pharmaceutical agents such as pharmacologically active peptides and proteins have been mainly administered by intravenous or subcutaneous injections. However, administering pharmaceutical agents by injections causes great psychological strain and physical pain to patients and also has a problem in terms of inconvenience in that self-administration by patients is difficult. Therefore in recent years, methods of administering drugs via a mucosal route such as through pulmonary mucosa, nasal mucosa, oral mucosa, vaginal mucosa, gastric mucosa, gastrointestinal mucosa and like mucosae have been attracting attention.

However, since transmucosal absorbability of peptides and proteins is generally low, it is not always easy for peptides and proteins to be absorbed through the mucosa in an amount sufficient to achieve the intended pharmaceutical effects. Therefore, various absorption enhancers for efficient transmucosal absorption of peptides and proteins have been developed. For examples, methods using, as an absorption enhancer, a cytidine nucleotide derivative (Japanese Patent Unexamined Patent Publication No. 1994-9424) or chitosan having a molecular weight of 5000 or more (Japanese Patent Unexamined Patent Publication No. 1999-116499) have been reported.

Although various methods as mentioned above have been proposed, the development of a technique enabling mucosal absorption by a more practical, effective method is still awaited as a means for improving the absorption of peptides and proteins that are difficult to absorb through the mucosa.

### DISCLOSURE OF THE INVENTION

An object of the invention is to provide a composition for transmucosal administration that enables pharmacologically active peptides and proteins to be efficiently absorbed through the mucosa such as pulmonary mucosa, nasal mucosa, oral mucosa, vaginal mucosa, gastric mucosa, gastrointestinal mucosa or the like. An another object of the invention is to provide a method which efficiently enhances the transmucosal absorption of pharmacologically active peptides and proteins.

To achieve the above object, the present inventors carried out extensive research. As a result, the inventors found that when a composition for transmucosal administration contains a chitosan oligosaccharide having a polymerization degree of from 2 to 20, a derivative thereof, or glucosamine, together with a pharmacologically active peptide or protein, transmucosal absorption of the pharmacologically active peptide or protein is enhanced, thus providing a composition capable of exhibiting excellent pharmacological effects when applied to the mucosa. The present invention has been accomplished based on the above finding.

The invention provides the following compositions for transmucosal administration:
Item 1. A composition for transmucosal administration comprising the following components (i) and (ii):
   (i) at least one member selected from the group consisting of peptides and proteins having pharmacological activity; and
   (ii) at least one member selected from the group consisting of chitosan oligosaccharides having a polymerization degree of from 2 to 20, derivatives thereof, glucosamine, and salts thereof.
Item 2. A composition according to Item 1 wherein component (i) is at least one member selected from the group consisting of antibiotics, hematopoietics, therapeutic agents for infectious diseases, antidementia agents, antiviral agents, antitumor agents, antipyretics, analgesics, antiphlogistics, antiulcer agents, antiallergic agents, antidepressants, psychotropic agents, cardiotonic agents, antiarrhythmics, vasodilators, hypotensive agents, therapeutic agents for diabetes, anticoagulants, cholesterol depressors, therapeutic agents for osteoporosis, hormones and vaccines.
Item 3. A composition according to Item 1 wherein component (i) is at least one member selected from the group consisting of cytokines, peptide hormones, growth factors, factors that act on the cardiovascular system, cell-adhesion factors, factors that act on the central or peripheral nervous systems, factors that act on body fluid electrolytes and organic substances in blood, factors that act on bones and the skeleton, factors that act on the digestive system, factors that act on the kidney and urinary systems, factors that act on connective tissues and the skin, factors that act on sensory organs, factors that act on the immune system, factors that act on the respiratory system, factors that act on the reproductive system, and enzymes.
Item 4. A composition according to Item 1 wherein component (i) is at least one member selected from the group consisting of interferons, interleukins, insulins, growth hormones, calcitonins, luteinizing hormone releasing hormones, adrenocortical hormones, luteinizing hormones, parathyroid hormones, and active fragments of parathyroid hormones.
Item 5. A composition according to Item 1 wherein component (ii) is at least one member selected from the group consisting of chitosan oligosaccharides having a polymerization degree of from 2 to 15, derivatives thereof, glucosamine, and salts thereof.
Item 6. A composition according to Item 1 comprising component (ii) in a proportion of 0.001 to 1 x 10⁶ parts by weight per 100 parts by weight of component (i).
Item 7. A composition according to Item 1 which is a liquid composition comprising component (i) in a proportion of 1 × 10⁻⁶ to 30 w/v%.
Item 8. A composition according to Item 1 which is a liquid composition comprising component (ii) in a proportion of 0.01 to 30 w/v%.
Item 9. A composition according to Item 1 which is a solid composition comprising component (i) in a proportion of 1 × 10⁻⁵ to 99 % by weight.
Item 10. A composition according to Item 1 which is a solid composition comprising component (ii) in a proportion of 0.1 to 50 % by weight.
Item 11. A composition according to Item 1 which is a composition for transnasal, transgastrointestinal, transpulmonary, oral mucosal, ocular mucosal or transvaginal mucosal administration.

The invention also provides the following methods of enhancing the transmucosal absorption:
Item 12. A method of enhancing, in a mammal, the transmucosal absorption of at least one member selected from the group consisting of peptides and proteins having pharmacological activity,
   the method comprising co-administering to the mucosa of said mammal (ii) at least one member selected from the group consisting of chitosan oligosaccharides having a polymerization degree of from 2 to 20, derivatives thereof, glucosamine, and salts thereof with (i) at least one member selected from the group consisting of peptides and proteins having pharmacological activity.
Item 13. A method according to Item 12 wherein component (i) is at least one member selected from the group consisting of antibiotics, hematopoietics, therapeutic agents for infectious diseases, antidementia agents, antiviral agents, antitumor agents, antipyretics, analgesics, antiphlogistics, antiulcer agents, antiallergic agents, antidepressants, psychotropic agents, cardiotonic agents, antiarrhythmics, vasodilators, hypotensive agents, therapeutic agents for diabetes, anticoagulants, cholesterol depressors, therapeutic agents for osteoporosis, hormones and vaccines.
Item 14. A method according to Item 12 wherein component (i) is at least one member selected from the group consisting of cytokines, peptide hormones, growth factors, factors that act on the cardiovascular system, cell-adhesion factors, factors that act on the central or peripheral nervous systems, factors that act on body fluid electrolytes and organic substances in blood, factors that act on bones and the skeleton, factors that act on the digestive system, factors that act on the kidney and urinary systems, factors that act on connective tissues and the skin, factors that act on sensory organs, factors that act on the immune system, factors that act on the respiratory system, factors that act on the reproductive system, and enzymes.
Item 15. A method according to Item 12 wherein component (i) is at least one member selected from the group consisting of interferons, interleukins, insulins, growth hormones, calcitonins, luteinizing hormone releasing hormones, adrenocortical hormones, luteinizing hormones, parathyroid hormones, and active fragments of parathyroid hormones.
Item 16. A method according to Item 12 wherein component (ii) is at least one member selected from the group consisting of chitosan oligosaccharides having a polymerization degree of from 2 to 15, derivatives thereof, glucosamine, and salts thereof.
Item 17. A method according to Item 12 comprising co-administering component (ii) is administered with component (i) in a proportion of 0.001 to 1 x 10⁶ parts by weight per 100 parts by weight of component (i).
Item 18. A method according to Item 12 comprising administering a liquid composition comprising components (i) and (ii), the liquid composition containing component (i) in a proportion of 1 × 10⁻⁶ to 30 w/v%.
Item 19. A method according to Item 12 comprising administering a liquid composition comprising components (i) and (ii), the liquid composition containing component (ii) in a proportion of 0.01 to 30 w/v%.
Item 20. A method according to Item 12 comprising administering a solid composition comprising components (i) and (ii), the solid composition containing component (i) in a proportion of 1 × 10⁻⁵ to 99 % by weight.
Item 21. A method according to Item 12 comprising administering a liquid composition comprising component (i) and (ii), the solid composition containing component (ii) in a proportion of 0.1 to 50 % by weight.
Item 22. A method according to Item 12 wherein the mucosa is nasal, gastrointestinal, pulmonary, oral, ocular or vaginal muosa. Item 23. A method according to Item 12, comprising administering the composition according to any one of Items 1 to 11 to the mucosa of said mammal.

### Further, the invention provides the following use:

Item 24. Use of (i) at least one member selected from the group consisting of peptides and proteins having pharmacological activity; and (ii) at least one member selected from the group consisting of chitosan oligosaccharides having a polymerization degree of from 2 to 20, derivatives thereof, glucosamine, and salts thereof, for producing a composition for transmucosal administration.

### BRIEF DESCRIPTION OF THE DRAWINGS

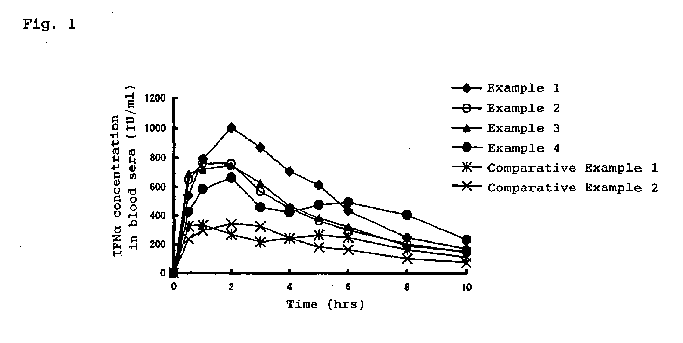

Fig. 1 shows the changes in average IFNα concentration in blood sera over time in Test Example 1 when the compositions (Examples 1 to 4 and Comparative Examples 1 to 2) were administered to the tracheae of rats.

### BEST MODE FOR CARRYING OUT THE INVENTION

The invention is described below in more detail.

### I. composition for transmucosal administration

### Component (i)

The composition for transmucosal administration of the present invention comprises as component (i) at least one member selected from the group consisting of peptides and proteins having physiological activity.

The peptides and proteins having physiological activity usable in present invention include peptides comprising two or more amino acids and derivatives thereof. Examples of such peptides and proteins include, in addition to those consisting only of amino acids, those modified with sugars, such as galactose, mannose, etc., or sugar chains; those modified with polyethylene glycol or other synthetic polymers or chondroitin sulfate, hyaluronic acid or other natural polymers; those modified with other non-peptidic compounds; etc. Also usable are hybrid peptides obtained by adding peptides with other functions to the amino acid sequences necessary for exhibiting physiological activity. In addition, fragments of known pharmacologically active peptides and proteins are also usable, as long as the fragments retain pharmacological activity.

The molecular weights of such peptides and proteins are not limited, and may be, for example, about 200 to about 200000, preferably about 200 to about 100000, and more preferably about 200 to about 50000.

Peptides and proteins for use in the present invention are not limited in their transmucosal absorption characteristics, and may have high or low transmucosal absorbability. As used herein, "high transmucosal absorbability" means that the peptide or protein is absorbed through the mucosa in a pharmaceutically effective amount when administered in a normal dose without using a transmucosal absorption enhancer, and "low transmucosal absorbability" means that the peptide or protein is not absorbed through the mucosa in a pharmaceutically effective amount when administered in a normal dose, unless the peptide or protein is used in combination with a transmucosal absorption enhancer. In view of an effect of the present invention, i.e., improvement of transmucosal absorbability of peptides and proteins, peptides and proteins with low transmucosal absorbability are advantageously used.

Such peptides and proteins may be natural, recombinantly produced, or chemically synthesized.

Peptides and proteins having physiological activity include, for example, components for use as antibiotics, hematopoietics, therapeutic agents for infectious diseases, antidementia agents, antiviral agents, antitumor agents, antipyretics, analgesics, antiphlogistics, antiulcer agents, antiallergic agents, antidepressants, psychotropic agents, cardiotonic agents, antiarrhythmics, vasodilators, hypotensive agents, therapeutic agents for diabetes, anticoagulants, cholesterol depressors, therapeutic agents for osteoporosis, hormones, and vaccines.

Examples of such peptides and proteins include cytokines, peptide hormones, growth factors, factors that act on the cardiovascular system, cell-adhesion factors, factors that act on the central or peripheral nervous systems, factors that act on body fluid electrolytes and organic substances in blood, factors that act on bones and the skeleton, factors that act on the digestive system, factors that act on the kidney and urinary system, factors that act on connective tissues and the skin, factors that act on sensory organs, factors that act on the immune system, factors that act on the respiratory system, factors that act on the reproductive system, and enzymes. Among these, preferable examples include cytokines, peptide hormones, growth factors, factors that act on the cardiovascular system, factors that act on the central or peripheral nervous systems, factors that act on body fluid electrolytes and organic substances in blood, factors that act on bones and the skeleton, factors that act on the digestive system, factors that act on the immune system, factors that act on the respiratory system, factors that act on the reproductive system, and enzymes.

The following are specific examples of such peptides and proteins. Peptides and proteins usable in the present invention are not limited to the following.
Cytokines: for example, interferons (interferons-α,β,γ), interleukins (interleukins-1 to -11), tumor necrosis factors (TNFs-α,β), malignant leukocyte inhibitory factors (LIFs), erythropoietins, granulocyte colony stimulating factors (G-CSFs), granulocyte-macrophage colony stimulating factors (GM-CSFs), macrophage colony stimulating factors (M-CSFs), thrombopoietins, platelet growth stimulating factors, megakaryocyte growth stimulating factors, etc.
Peptide hormones: for example, insulins, growth hormones, luteinizing hormone-releasing hormones (LH-RHs), adrenocorticotropic hormones (ACTHs), amylins, oxytocins, luteinizing hormones, etc.
Growth factors: for example, nerve growth factors (NGF, NGF-2/NT-3), epidermal growth factors (EGFs), fibroblast growth factors (FGFs), insulin-like growth factors (IGFs), transformation growth factors (TGFs), platelet-derived cell growth factors (PDGFs), hepatocyte growth factors (HGFs), etc.
Factors that act on the cardiovascular system: for example, endothelins, endothelin inhibitors, endothelin antagonists, endothelin producing enzyme inhibitors, vasopressins, renins, angiotensin I, angiotensin II, angiotensin III, angiotensin I inhibitors, angiotensin II receptor antagonists, atrial natriuretic polypeptides (ANPs), antiarrhythmic peptides, etc. Cell adhesion factors: for example, laminins, intercellular adhesion molecule I (ICAMs-1), etc.
Factors that act on the central and peripheral nervous systems: for example, enkephalins, endorphins, kyotorphins, neurotropic factors (NTFs), calcitonin gene-related peptides (CGRPs), adenylate cyclase activating peptides (PACAPs), thyroid hormone releasing hormones (TRHs), neurotensins, etc.
Factors that act on body fluid electrolytes and organic substances in blood: for example, calcitonins, apoprotein E, hirudins, etc.
Factors that act on bones and the skeleton: for example, thyroid hormones, active fragments of thyroid hormones, histone H4-related osteogenic growth peptides, etc.
Factors acting on the digestive system: for example, secretins, gastrins, etc.
Factors that act on the kidney and urinary system: for example, brain-derived natriuretic peptides, urotensins, etc.
Factors that act on sensory organs: for example, substance P and the like.
Factors that act on the immune system: for example, chemotactic peptides, bradykinins, etc.
Factors that act on the respiratory system: for example, factors that control asthmatic reactions, etc.

Among these, advantageously usable examples of component (i) include interferons, interleukins, insulins, growth hormones, luteinzing hormone releasing hormones, adrenocorticotropic hormones, luteinizing hormones, calcitonins, thyroid hormones, and active fragments of thyroid hormones. Particularly preferable examples include interferons.

The dosage amount per day of component (i) varies according to the type of component (i), the age and sex of the patient, and other conditions, and is usually about 0.001 to about 100 mg/day/adult, and preferably 0.01 to about 10 mg/day/adult.

### Component (ii)

The composition for transmucosal administration of the invention comprises as component (ii) at least one member selected from the group consisting of chitosan oligosaccharides having a polymerization degree of from 2 to 20, derivatives thereof, glucosamine, and salts thereof.

In the present invention, the term "chitosan oligosaccharide having a polymerization degree of from 2 to 20" means an oligosaccharide having 2 to 20 glucosamine residues linked by by β1-4 bonds.

In the invention, there is no limitation to the derivatives of chitosan oligosaccharides having a polymerization degree of from 2 to 20 insofar as the derivatives are pharmaceutically acceptable. Specific examples of such derivatives include carboxymethylates, carboxyethylates, hydroxyethylates, dihydroxypropylates, methylates, ethylates, glycolates, acylates, tosylates, sulfonates of the above-mentioned oligosaccharide, etc.

Moreover, there is no limitation to the salts of chitosan oligosaccharides having a polymerization degree of from 2 to 20, derivatives thereof, and glucosamine that are used as component (ii) insofar as they are pharmaceutically acceptable. Examples of such salts include salts formed with inorganic acids (e.g., hydrochloric acid, sulfuric acid, phosphoric acid, etc.); and salts formed with organic acids (e.g., acetic acid, tartaric acid, lactic acid, glutamic acid, maleic acid, alginic acid, citric acid, etc.).

Preferable examples of component (ii) are chitosan oligosaccharides having a polymerization degree of from 2 to 15, derivatives thereof, glucosamine, and salts thereof, and more preferable examples are chitosan oligosaccharides having a polymerization degree of from 2 to 10, derivatives thereof, glucosamine, and salts thereof.

The above-mentioned chitosan oligosaccharides used as component (ii) can be manufactured by hydrolyzing chitosan with hydrochloric acid or an enzyme (chitonase or the like). Alternatively, such chitosan oligosaccharides can be manufactured by hydrolyzing chitin with hydrochloric acid or an enzyme (chitinase or the like), and then deacetylating the same with a strong alkali solution.

### Composition for transmucosal administration

In the composition for transmucosal administration of the invention, there is no limitation to the proportion of the above-described component (i) to component (ii). Component (ii) is usually used in a proportion of 0.001 to 1×10⁶ parts by weight per 100 parts by weight of component (i), preferably 0.1 to 1×10⁵ parts by weight, and more preferably 0.1 to 1×10⁴ parts by weight. The absorption of component (i) can be remarkably enhanced by employing the above proportion.

The proportion of the above-mentioned component (i) in the composition of the invention may be any amount effective for demonstrating the pharmacological effect when applied to the mucosa, and be suitably determined according to gender and age of a patient, route of administration, composition form, dosage form, type and daily dosage of component (i) used, type of component (ii) used, intended pharmacological effect, etc. For example, the proportion of component (i) in the composition is 1×10⁻⁷ to 99% by weight per the total weight of the composition. Specifically, when the composition of the invention is taken in the form of a liquid, the proportion of component (i) is usually 1×10⁻⁶ to 30 w/v%, preferably 1×10⁻⁵ to 10 w/v%, and more preferably 1×10⁻⁴ to 1 w/v%. The unit "w/v%" is equivalent to the unit "g/100ml". When the composition of the invention is taken in the form of a solid, the proportion of component (i) is usually 1×10⁻⁵ to 99% by weight, preferably 1×10⁻⁴ to 50% by weight, and more preferably 1×10⁻³ to 10% by weight per the total weight of the composition.

The proportion of the above-described component (ii) may be any amount effective for enhancing the transmucosal absorption of component (i), and be suitably determined based on the above-mentioned proportion of component (ii) to component (i) and the content of component (i). For example, the proportion of component (ii) in the composition is 0.001 to 50% by weight per the total weight of the composition. Specifically, when the composition of the invention is taken in the form of a liquid, the content of component (ii) is usually 0.01 to 30 w/v%, preferably 0.1 to 10 w/v%, and more preferably 0.5 to 5 w/v%. When the composition of the invention is taken in the form of a solid, component (ii) is usually used in a proportion of 0.1 to 10% by weight, preferably 0.5 to 30% by weight, and more preferably 1 to 10% by weight per the total weight of the composition.

In addition to components (i) and (ii), the composition of the invention may contain various medicinal ingredients that are typically contained in the composition for transmucosal administration.

Moreover, in addition to the above-mentioned components, the composition for transmucosal administration of the invention may suitably contain carriers or additives that are generally contained in a composition for transmucosal administration according to the intended use or form of the composition. The amounts of carriers or additives may be suitably determined according to the ranges ordinarily used in this field. Such carriers or additives that can be used are not limited, and specific examples thereof include various carriers such as water, physiological saline, other aqueous solvents, aqueous bases, or oily bases; various additives such as excipients, binders, pH modifiers, disintegrators, absorption promoters, lubricants, colorants, corrigents, flavorings, etc.

Specific examples of such additives include lactose, saccharose, mannitol, sodium chloride, glucose, calcium carbonate, kaolin, crystalline cellulose, silicates and other excipients; water, ethanol, simple syrup, glucose solutions, starch solutions, gelatin solutions, carboxymethylcellulose, sodium carboxymethylcellulose, shellac, methylcellulose, hydroxypropylmethylcellulose, hydroxypropylcellulose, polyvinylpyrrolidone, polyvinyl alcohol, gelatin, dextrin, pullulan, and other binders; citric acid, citric anhydride, sodium citrate, sodium citrate dihydrate, anhydrous sodium monohydrogenphosphate, anhydrous sodium dihydrogenphosphate, sodium hydrogenphosphate, sodium dihydrogenphosphate and other pH modifiers; carmellose calcium, low-substituted hydroxypropylcellulose, carmellose, croscarmellose sodium, carboxymethyl starch sodium, crospovidone, polysorbate-80, and other disintegrators; quaternary ammonium bases, sodium lauryl sulfate, and other absorption promoters; purified talc, stearic acid salts, polyethylene glycol, colloidal silicic acid, sucrose esters of fatty acids, and other lubricants; yellow iron oxide, yellow iron sesquioxide, iron sesquioxide, β-carotene, titanium oxide, food colors (e.g., Food Blue No. 1), copper chlorophyll, riboflavin, and other colorants; ascorbic acid, aspartame, *Hydrangeae Dulcis Folium,* sodium chloride, fructose, saccharin, powdered sugar, and other corrigents; etc.

In addition to the above-mentioned components, the composition of the invention may further contain biodegradable polymers as a base. Typical examples of such biodegradable polymers include polylactic acid, poly (lactic acid-glycolic acid) copolymers, polyhydroxy butyric acid, poly (hydroxybutyric acid-glycolic acid) copolymers, mixtures thereof, etc, but usable biodegradable polymers are not limited thereto.

The composition of the invention can take any form insofar as it can be applied to mucosa, and can be used in the form of a solid, liquid, semi-solid, suspension, powder, or fine particles. Moreover, the composition of the invention may be formed into particles such as microcapsules (microsperes), etc. according to known procedures.

The composition of the invention can be applied to any mucosa. Examples of mucosa to which the composition of the invention is applicable include nasal mucosa, digestive mucosa (gastrointestinal mucous), lung mucosa, pulmonary mucosa, vaginal mucosa, oral mucosa, ocular mucosa, tracheal mucosa, etc. Among the above, nasal mucosa, digestive mucosa, lung mucosa, and vaginal mucosa are preferable in view of patient's compliance, etc.

The composition of the invention can be formulated into various dosage forms according to the composition form or the target mucosa. The composition of the invention can be used as a pharmaceutical composition and administered to the mucosa of a patient by a suitable rout of administration according to the target mucosa and the dosage form.

### II. Method of enhancing the transmucosal absorption

As described above, component (ii) can enhance the transmucosal absorption of pharmacologically active peptides and proteins. Thus the present invention provides a method of enhancing the transmucosal absorption of at least one member selected from the group consisting of peptides and proteins having pharmacological activity. The method is carried out by co-administering to the mucosa of human or other mammals (ii) at least one member selected from the group consisting of chitosan oligosaccharides having a polymerization degree of from 2 to 20, derivatives thereof, glucosamine, and salts thereof with (i) at least one member selected from the group consisting of peptides and proteins having pharmacological activity.

In the method, the kind and dose of component (i), the kind and dose of component (ii), the ratio of component (ii) to component (i), target mucosa, etc. may be the same as described above in the "I. Composition for transmucosal administration".

The method is preferably carried out by administrating the composition for transmucosal administration to the mucosa of a mammal.

### Examples

Hereafter, the invention is described in detail according to examples and experimental examples, but is not limited thereto. The average molecular weight of chitosan used in Example 4 and Comparative Example 1 was obtained by GPC (Gel Permeation Chromatography) mode using high performance liquid chromatography (HPLC) as described in "Chitin and Chitosan handbook", edited by Chitin and Chitosan Study Group, published by Gihodo Shuppan, in Chapter 8, Method for analyzing chitin and chitosan.

### Example 1

A chitosan hexamer in which 6 glucosamine residues are linked by β1-4 bonds (product name: Chitosan Hexamer, molecular weight: 1204, Code No.400436, purchased from Seikagaku Corporation) was dissolved in water for injection (product number: 2D71N, sold by: Otsuka Pharmaceutical Co., Ltd.), preparing a 5 mg/mL chitosan hexamer solution. The thus-obtained solution (0.5 mL) was added to a vial containing 10 million IU of interferon α (IFNα, natural interferon α, product name: OIF, product number: 3G86F10, manufactured and sold by: Otsuka Pharmaceutical Co., Ltd.), and shaken gently to dissolve freeze-dried substances contained in the vial, preparing a composition having an IFNα concentration of 20 million IU/mL (about 0.01 w/v%).

### Example 2

A composition having an IFNα concentration of 20 million IU/mL (about 0.01 w/v%) was prepared in the same manner as in Example 1 except that chitosan dimer in which two glucosamine residues are linked by a β1-4 bond (product name: Chitosan Dimer, molecular weight: 413, Code No.400432, purchased from Seikagaku Corporation) was used in the same weight instead of the chitosan hexamer.

### Example 3

A composition having an IFNα concentration of 20 million IU/mL (about 0.01 w/v%) was prepared in the same manner as in Example 1 except that D-(+)-glucosamine (molecular weight: 216, Code No.101782, purchased from Wako Pure Chemical Industries, Ltd.) was used in the same weight instead of the chitosan hexamer.

### Example 4

A composition having an IFNα concentration of 20 million IU/mL (about 0.01 w/v%) was prepared in the same manner as in Example 1 except that chitosan oligosaccharide (product name: Water-soluble chitosan, average molecular weight: 1800, product No. 037-14303, purchased from Wako Pure Chemical Industries, Ltd.) was used in the same weight instead of the chitosan hexamer.

### Example 5

A chitosan hexamer in which 6 glucosamine residues are linked by β1-4 bonds (product name: Chitosan Hexamer, molecular weight: 1204, Code No.400436, purchased from Seikagaku Corporation) was dissolved in an isotonic phosphate buffer, preparing a 5 mg/mL chitosan hexamer solution. Salmon calcitonin (Code No.T3660, purchased from Sigma-Aldrich Japan) was dissolved in an isotonic phosphate buffer, preparing a 10 µg/mL salmon calcitonin solution. The thus-obtained chitosan hexamer solution (0.5 mL) and salmon calcitonin solution(0.5 mL) ware mixed, preparing a composition having a salmon calcitonin concentration of 1 µg/mL.

### Comparative Example 1

A composition having an IFNα concentration of 20 million IU/mL (about 0.01 w/v%) was prepared in the same manner as in Example 1 except that chitosan (product name: CHITOSAN EF, average molecular weight: 48000, LOT 0302190, purchased from NOF CORPORATION) was used in the same weight instead of the chitosan hexamer.

### Comparative Example 2

A composition having an IFNα concentration of 20 million IU/mL (about 0.01 w/v%) was prepared in the same manner as in Example 1 except that no chitosan hexamer was used.

### Comparative Example 3

A composition having a salmon calcitonin concentration of 1 µg/mL was prepared in the same manner as in Example 5 except that no chitosan hexamer was used.

### Experimental Example 1

In order to evaluate the mucosal absorbability of the compositions of Examples 1 to 4 and Comparative Examples 1 and 2, and in particular absorption characteristics through the lung mucosa, the following tests were conducted.

Wistar male rats (body weight: about 200 g) which had been deprived of food for 18 hours were used as test animals. Each rat was immobilized under isoflurane anesthetization, and a Teflon tube (internal diameter of 0.8 mm and external diameter of 1.6 mm) was inserted to a depth of about 5 cm from the oral cavity to the trachea. Through this Teflon tube, 0.5 mL/kg of one of each of the compositions of Examples 1 to 4 and Comparative Examples 1 and 2 was administered into the rat tracheae. 0.2 mL of blood was collected from a subclavian vein 30 minutes, 1 hour, 2 hours, 3 hours, 4 hours, 5 hours, 6 hours, 8 hours, and 10 hours after the tracheal administration. The collected blood samples were subjected to centrifugation at 1800 g for 10 minutes, obtaining blood serum samples. IFNα concentrations of the thus-obtained serum samples were measured by an enzyme-labelled antibody technique (ELISA) using a human IFN measuring kit (manufactured and sold by: Japan Imunoresearch Laboratories, Co., Ltd.).

Table 1 and Figure 1 show the results. Table 1 shows the changes in average IFNα concentration in blood sera over time (n=4). Table 1 shows the mean values of pharmacokinetic parameters (n=4). Note that the parameters in Table 1 have the following meanings:
AUC₁₀ₕᵣ: Area under the blood concentration - time curve up to 10 hours after the administration (IU·hr/mL)
AUCinf: Area under the blood concentration - time curve up to infinite time (IU · hr/mL)
Cₘₐₓ: Maximum IFNα concentration in blood (IU/mL)
Tₘₐₓ: Time to reach the maximum IFNα concentration in blood (hr)

As is clear from the results, in the compositions (Examples 1 to 4) comprising a chitosan hexamer, chitosan dimer, chitosan oligosaccharide of a molecular weight of 1800, or glucosamine together with IFNα, the maximum IFNα concentrations in blood (Cₘₐₓ) and the Serum IFNα concentrations up to 10 hours after the administration-area under the blood concentration time curve (AUC₁₀ₕᵣ) were increased, compared to a composition comprising just chitosan (molecular weight of 48000, Comparative Example 1) or a composition comprising just IFNα (Comparative Example 2).

**Table 1**

| | AUC₁₀ₕᵣ (IU·hr/mL) | Cmax (IU/mL) | Tmax (hr) | AUC_{∞} (IU·hr/mL) |
|---|---|---|---|---|
| Example 1 | 5385 | 1006 | 2.0 | 6105 |
| Example 2 | 4050 | 817 | 1.5 | 4871 |
| Example 3 | 4131 | 768 | 1.8 | 4967 |
| Example 4 | 4464 | 670 | 2.8 | 5899 |
| Com. Ex. 1 | 2227 | 356 | 3.1 | 2812 |
| Com. Ex. 2 | 1968 | 375 | 1.9 | 2373 |

From these results, it is clear that, by adding a chitosan oligosaccharide having a polymerization degree of from 2 to 20 or glucosamine itself, together with a pharmacologically active peptide or protein, the transmucosal absorption of the biologically active peptide or protein contained in the composition can be increased, and the bioavailability thereof can be significantly enhanced.

### Experimental Example 2

In order to evaluate the mucosal absorbability of the compositions of Example 5 and Comparative Example 3, and in particular absorption characteristics through the lung mucosa, the following tests were conducted.

Wistar male rats (body weight: about 200g) which had been deprived of food for 18 hours were used as test animals. Each rat was immobilized under isoflurane anesthetization, and 0.1 mL of one of each of the compositions of Example 5 and Comparative Example 3 was administered into the rat tracheae by MicroSprayer^{™} (Penn-century, Inc.). 0.2 mL of blood was collected from a subclavian vein 10 minutes, 30 minutes, 1 hour, 2 hours, 3 hours, 4 hours, 5 hours, and 6 hours after the tracheal administration. Calcium concentrations of the thus-obtained serum samples were measured by a methylxylenol blue (MXB) method using a calcium measuring kit (manufactured and sold by: Wako Pure Chemical Industries, Ltd.).

The results show that the composition (Example 5) comprising a chitosan hexamer together with salmon calcitonin has the serum calcium level reduction effect 3.8 times that of the composition (Comparative Example 3) comprising just salmon calcitonin, and the effect obtained by administering the composition of Example 5 was maintained for at least 6 hours after the administration.

### INDUSTRIAL APPLICABILITY

Since the composition for transmucosal administration of the invention contains chitosan oligosaccharides having a polymerization degree of from 2 to 20 with a molecular weight of 3000 or less, derivatives thereof, glucosamine or salts thereof together with a peptide and/or protein with physiologically activity, the mucosal absorbability of peptide and/or protein having pharmacological activity are enhanced. Therefore, the composition for transmucosal administration of the invention can efficiently demonstrate medicinal action based on the peptide and/or protein having pharmacological activity.

Moreover, the composition for transmucosal administration of the invention is extremely useful as a preparation for mucosal administration of a physiologically active peptide and/or protein that needs to be repeatedly administered over the long term because self-administration thereof to the mucosa, such as nasal mucosa, lung mucosa, vaginal mucosa, digestive mucosa, etc., is possible, unlike injections which cause pain to the patient.

## Claims

1. A composition for transmucosal administration comprising the following components (i) and (ii):
(i) at least one member selected from the group consisting of peptides and proteins having pharmacological activity; and
(ii) at least one member selected from the group consisting of chitosan oligosaccharides having a polymerization degree of from 2 to 20, derivatives thereof, glucosamine, and salts thereof.

2. A composition according to claim 1 wherein component (i) is at least one member selected from the group consisting of antibiotics, hematopoietics, therapeutic agents for infectious diseases, antidementia agents, antiviral agents, antitumor agents, antipyretics, analgesics, antiphlogistics, antiulcer agents, antiallergic agents, antidepressants, psychotropic agents, cardiotonic agents, antiarrhythmics, vasodilators, hypotensive agents, therapeutic agents for diabetes, anticoagulants, cholesterol depressors, therapeutic agents for osteoporosis, hormones and vaccines.

3. A composition according to claim 1 wherein component (i) is at least one member selected from the group consisting of cytokines, peptide hormones, growth factors, factors that act on the cardiovascular system, cell-adhesion factors, factors that act on the central or peripheral nervous systems, factors that act on body fluid electrolytes and organic substances in blood, factors that act on bones and the skeleton, factors that act on the digestive system, factors that act on the kidney and urinary systems, factors that act on connective tissues and the skin, factors that act on sensory organs, factors that act on the immune system, factors that act on the respiratory system, factors that act on the reproductive system, and enzymes.

4. A composition according to claim 1 wherein component (i) is at least one member selected from the group consisting of interferons, interleukins, insulins, growth hormones, calcitonins, luteinizing hormone releasing hormones, adrenocortical hormones, luteinizing hormones, parathyroid hormones, and active fragments of parathyroid hormones.

5. A composition according to claim 1 wherein component (ii) is at least one member selected from the group consisting of chitosan oligosaccharides having a polymerization degree of from 2 to 15, derivatives thereof, glucosamine, and salts thereof.

6. A composition according to claim 1 comprising component (ii) in a proportion of 0.001 to 1 x 10⁶ parts by weight per 100 parts by weight of component (i).

7. A composition according to claim 1 which is a liquid composition comprising component (i) in a proportion of 1 x 10⁻⁶ to 30 w/v%.

8. A composition according to claim 1 which is a liquid composition comprising component (ii) in a proportion of 0.01 to 30 w/v%.

9. A composition according to claim 1 which is a solid composition comprising component (i) in a proportion of 1 × 10⁻⁵ to 99 % by weight.

10. A composition according to claim 1 which is a solid composition comprising component (ii) in a proportion of 0.1 to 50 % by weight.

11. A composition according to claim 1 which is a composition for transnasal, transgastrointestinal, transpulmonary, oral mucosal, ocular mucosal or transvaginal mucosal administration.

12. A method of enhancing, in a mammal, the transmucosal absorption of at least one member selected from the group consisting of peptides and proteins having pharmacological activity, the method comprising co-administering to the mucosa of said mammal (ii) at least one member selected from the group consisting of chitosan oligosaccharides having a polymerization degree of from 2 to 20, derivatives thereof, glucosamine, and salts thereof with (i) at least one member selected from the group consisting of peptides and proteins having pharmacological activity.

13. A method according to claim 12, comprising administering to the mucosa of said mammal the composition according to any one of claims 1 to 11.

14. Use of (i) at least one member selected from the group consisting of peptides and proteins having pharmacological activity; and(ii) at least one member selected from the group consisting of chitosan oligosaccharides having a polymerization degree of from 2 to 20, derivatives thereof, glucosamine, and salts thereof, for producing a composition for transmucosal administration.
